# EUROPEAN PATENT SPECIFICATION

(11) **EP 0 795 332 B1**
(45) Date of publication and mention of the grant of the patent: **01.06.2005**
(21) Application number: 97104000.1
(22) Date of filing: 11.03.1997
(51) Int. Cl.: A61K 38/21, A61K 31/57

(54) **Medical use of gamma-interferon in interstitial lung diseases**
Medizinische Verwendung von Gamma-Interferon im interstitiellen Lungenerkrankungen
Utilisation médicale de gamma interféron dans les maladies pulmonaires interstitielles

(30) Priority: 14.03.1996 DE 96104021
(43) Date of publication of application: 17.09.1997
(73) Proprietor: Mondobiotech Interferon SA, 6925 Gentilino (CH)
(72) Inventor: Block, Lutz Henning, Prof. Dr., 79312 Emmendingen (DE); Ziesche, Rolf, Dr., 1210 Wien (AT)
(74) Representative: Salgo, Reinhold Caspar, Dr.

(56) References cited:
- EP-A- 0 328 255
- WO-A-87/07842
- WO-A-89/01341
- GRANSTEIN R D ET AL: "THE SYSTEMIC ADMINISTRATION OF GAMMA INTERFERON INHIBITS COLLAGEN SYNTHESIS AND ACUTE INFLAMMATION IN A MURINE SKIN WOUNDING MODEL" JOURNAL OF INVESTIGATIVE DERMATOLOGY, vol. 93, no. 1, July 1989, pages 18-27, XP000197306
- ZABEL P: "PATHOGENESE INTERSTITIELLER LUNGEN-VERANDERUNGEN BEI KOLLAGENOSEN -FOLGERUNGEN FUR DIE THERAPIE PATHOGENESIS OF THE INTERSTITIAL PULMONARY MANIFESTATIONS IN COLLAGEN VASCULAR DISEASES - THERAPEUTIC IMPLICATIONS" IMMUNITAET UND INFEKTION, vol. 23, no. 3, 1 January 1995, pages 97-106, XP000563029

## Description

The present invention relates to a novel medical use of interferon-γ. This invention describes that interferon-γ can be used for the treatment of all forms of interstitial lung diseases. The invention reveals in addition that, above all, a combinatorial administration of interferon-γ together with a glucocorticoid may be used successfully even for the long-term therapy of said disease.

### Background of the Invention

Interstitial lung diseases are a heterogenetic group of chronic inflammations of the lung (also named Interstitial Pulmonary Fibrosis, IPF). Different forms of IPF are known which are, for example, idiopathic fibrosis, exogen allergic alveolitis, sarcoidosis or scleroderma. They are best desribed by a chronic inflammatory process of the alveolar wall and the surrounding terminal bronchioli leading to a progressive destruction of the gas-exchanging membranes of the lung. As consequence regluar lung tissue is replaced by smooth muscle cells and interstitial matrix protein fibres mainly consisting of collagen. In contrast to many wound healing processes of the lung, IPF is not self-limiting. Moreover, none of the features held to be characteristic for interstitial lung diseases are pathognomonic. Except for about 1% of all forms of IPF the etiology is completely unknown. Although a lot of attempts were made in the past activation, attraction and proliferation mechanisms of immunocompetent cells concerned (such as lymphocytes and macrophages) leading to the inflammatory processes during the disease, remain thoroughly unclear. Additionally, the unclear situation is intensified by the observation that IPF can be associated with or partly mimick "connective tissue diseases" (at least the involvement of the lung) like rheumatoid arthritis (e.g. Popper et al., 1972, Chest 62, 243), systemic sclerosis (e.g. Weaver et al., 1967, Mayo Clin. Proc. 42, 754), lupus erythematodes (e.g. Turner-Stoles et al., 1982, Clin. Rheum. Dis. 8, 229), dermatomyositis (e.g. Furgusson et al., 1983, Thorax 38, 71), and Sjögren's syndrome (e.g. Liebow et al:, 1973, Med. Clin. N. Am. 57, 809). Moreover, certain drugs such as bleomycin and mineral dusts such as definite kinds of asbestos may cause symptoms of IPF.

As seen in acute phase of the disease the most common and probably the earliest event is a vasculitis leading to formation of interstitial edema and attraction of immunocompetent cells which subsequently also sequestrate into the alveolar space where they can be found e.g. by bronchoalveolar lavage. Nearly all sorts of inflammatory cells can be found during the development of an interstitial pneumonitis. However, it is striking that following the acute onset of the inflammatory process more and more lymphocytes and, afterwards, an increasing population of neutrophil (and a small portion of eosinophil) granulocytes can be observed within the tissue. Thus, it seems that a chronic activation of lymphocytes may play, at least in part, a certain role in IPF as well as in the chronic lymphocytic inflammation in the bronchial tissues of asthmatics.

Recently, it has been shown that the fibrotic response in chronic interstitial lung disease is controlled *in vitro* by a network of cytokines like interleukin-1, interleukin-6, tumor necrosis factor α and interferon-γ which all were produced by mononuclear cells like macrophages and lymphocytes (Wewers at al., 1984, J. Clin. Invest. 74, 2208). Moreover, it has been shown that interferon-γ is released *in vivo* in different forms of fibrosing alveolitis regardless of the lymphocyte subpopulations found in the bronchoalveolar lavage (Robinson et al., 1990, Thorax 45, 105). Furthermore, addition of interferon-γ to either cytokine results in a dose-dependent inhibition of fibroblast proliferation and collagen synthesis (Elias et al., 1990, Chest 97, 1439). This inhibition was, at least partly, mediated by fibroblast prostaglandin production induced by interleukin-1 or TNFα. The antiproliferative effect of interferon-γ has recently been confirmed in smooth muscle cells (Desmouliere et al., 1992, Exp. Cell Res. 201, 64). Numerous in vitro and in vivo studies have now presented striking evidence for a pathophysiological role of transforming growth factor-β (TGF-β) in IPF (e.g. Bienkowski and Gotkin, 1995, Proc. Soc. Experim. Biol. Med. 209, 118).In the bleomycin-induced model of lung fibrosis, interferon-γ effectively downregulated the transcription of TGF-β followed by a decrease of the transcription of procollagen I and III (Gurujeyalakshmi and Giri, 1995, Exp. Lung Res. 21, 791).

Interferon-γ is a naturally glycoprotein having a molucular weight of about 17 kD which can be commercially produced today also by recombinant techniques. Together with (other) cytokines it plays, as already discussed above, an important role within the human immune system, usually showing immunregulatory effects (that means an immunstimulating or an immunsuppressive effect dependent on cell activation and location). However, there are still outstandimg mysteries about interferons-γ' s complete and real role. What is sure is that it is made in the organism by at least three types of immune system cells (CD4 T helper 1, natural killer, CD8 cytotoxic suppressor) and has effects on at least four different cell types (CD4 T helper 2, macrophage, natural killer, B cell).

As pharmaceutical compound it is used nowadays with a certain success, above all, against some viral infections and tumors. Interferon-γ is usually applicable via parenteral, preferably via subcutaneous, injection. Maximum serum concentrations have been found after seven hours, half life in plasma is six hours. The main adverse effects consist of fever, chills, sweating, headache, myalgia and drowsiness.

These effects have been observed within the first hours after injection. Rare side effects are local pain and erythema, elevation of liver enzymes, reversable granulo- and thrombopenia and cardiotoxicity. No specific antibodies against recombinant interferon-γ have been observed up to now.

Despite their different origins interstitial lung diseases show a very uniform reaction in regard to the assessment of lung function. In terms of functional staging, three different phases can be distinguished: (1) illness-related diversity of the inflammation mainly due to the underlying immune reactions, (2) uniform, organ-specific inflammation with a slow but steady decrease of lung function, and (3) end-stage fibrosis with a general lack of reactivity regarding anti-inflammatory therapy. Phase (1) is characterized by a sometimes dramatic onset with fever, coughing, severe dyspnoea under exercise conditions or even at rest and (infrequently) hemoptysis as seen, for example, in allergic alveolitis, Goodpasture's syndrome, lupus erythematosus, acute sarcoidosis or Hamman-Rich syndrome. The disease often shows a rather slow development within the first months. It starts with breathlessness, either under conditions of physical exercise or at rest. Lung function shows a diminished total lung capacity due to the decrease of both inspiratory vital capacity and residual lung volume. The pulmonary gas exchange is severely diminished as seen in the decrease of carbon monoxide diffusing capacity and blood oxygen content under exercise conditions. With the ongoing inflammatory process, roentgenologic and functional findings show a more and more uniform pattern. The next years are almost characterized by a steady decrease of lung function and life quality, interrupted by episodes of acute inflammation, in most cases due to bacterial and viral infections which require glucocorticoid and antibiotic treatment. After approximately 5- 8 years lung tissue has been widely replaced by fibrotic tissue.

Interstitial lung disease cannot be cured until now. Drugs such as glucocorticosteroids or immunsuppresive drugs like azathioprin, cyclophosphamide or cyclosporins or combinations of them can improve the the symptoms and life quality only.

The most effective treatment is still the use of glucocorticoids, e.g. prednisolone. The typical regimen starts usually with an initial administration of 50 - 100 mg prednisolone or an equivalent thereof followed by a tapering of the dose to 10 - 20 mg per day. A maintenance dosage of 10 - 15 mg glucocorticoid per day must be administered for at least 6 months, in most of the cases for several years. Nevertheless, even long-term treatment with 10 mg prednisolone daily does not prevent deterioration of lung function whereas discontinuance of therapy is often impossible so that the course of treatment in general consists of alternating phases with lower and higher dosages. Despite continuous application the overall outcome of this prior art regimen is poor. Comprehensive studies show that the median survival time lies between four and five years calculated from the time when the first symptoms are reported. The overall survival time is about 16 years.

The prior art alternatives of treating interstitial lung diseases are, as described above, evidently limited, and thus, it is an object of the present invention to provide drugs and uses of them for medical treatment which lead to significant improvements for patients suffering from IPF.

### Summary of the invention

The present invention describes the manufacture of a medicament for use and a possibility of an improved method for treatment of interstitial lung diseases which shows significant advantages in contrast to the traditional treatment with glucocorticoids or immunosuppressive drugs alone or in combination. The invention shows that - using the drugs mentioned above and below - the symptoms of the disease can be distinctly reduced or mitigated, respectively, the life quality improved, and the survival time prolonged. This can be achieved by using interferon-γ alone or, what is preferred according to the invention, in combination with glucocorticoids, preferably prednisolone.
Thus, it is an object of the present invention to provide a new use of interferon-γ for the manufacture of a medicament, a pharmaceutical composition or a kit-of-parts for the treatment of interstitial lung diseases.

It is, furthermore, object of the invention to provide a new combinatorial treatment of interferon-γ and glucocorticoids. The invention relates, therefore, to the use of interferon-γ together with glucocorticoids for manufacturing a medicament, a pharmaceutical composition or a kit-of-parts for the treatment of interstitial lung diseases.

The combinatorial therapy of interferon-γ and glucocorticoids is especially advantageous.

According to the invention said drugs can be used successfully in acute as well as in long-term therapy of all forms of interstitial lung diseases. Hence, the invention relates to the use of interferon-γ, optionally together with glucocorticoids, for manufacturing a medicament, a pharmaceutical composition or a kit-of-parts even for the long-term treatment of all forms of interstitial lung diseases or interstitial pulmonary fibrosis, respectively.

Although it is known that interferon-γ may inhibit under certain circumstances the proliferation of fibroblasts (see above) there is no hint in the prior art that it was ever tried to apply it in the treatment of IPF. Interestingly, as it is shown in the present invention, the known amounts of interferon-γ which are generally used in the treatment of viral infections or tumors (about 25 - 100 µg / single dose, 2 - 3 times a week) have no or no significant effect in the treatment of interstitial lung diseases according to this invention. Remarkable therapeutical effects can be observed only when the interferon dosage is increased distinctly up to more than I00 µg / dose (2 - 3 times a week), preferably up to 200 µg / dose and more during a long-term administration.
Therefore, it is a further object of the invention to establish said novel use of interferon-γ, wherein the single dose of interferon-γ within said medicament varies from 1.0 to 3.5 µg / kg, preferably from 2.0 to 3.0 µg / kg.

It is another object of the invention to establish said new combinatorial treatment of interferon-γ and glucocorticoids, wherein the single dose of the glucocorticoids varies from 100 - 350 µg / kg, preferably from 100 - 150 µg /kg.

According to this invention the dosage of interferon-γ can be slightly diminished in the presence of glucocorticoids (in comparison with the application in absence of any glucocorticoid) in order to obtain the same therapeutical effect.

Because of that, undesired side effects of interferon-γ can be reduced. On the other hand, it could be possible that the presence of glucocorticoids slows down the side effects of interferon-γ. This could be the reason why it is possible according to the invention to use relatively high amounts of interferon during a long time of therapy without getting severe problems caused by side effects of interferon-γ when used alone according to the prior art.

Pharmaceutical compositions or medical kits-of parts comprising interferon-γ and glucocorticoids in concentrations and dosages as presented herein, are new.

It is, therefore, further object of the invention to provide a pharmaceutical composition comprising interferon-γ, at least one glucocorticoid and, optionally, a pharmaceutically acceptable carrier and/or excipients.

Finally, it is an object of the invention to provide a medical kit-of-parts suitable for the treatment of interstitial lung diseases comprising a first pharmaceutical composition comprising interferon-γ and a second pharmaceutical composition comprising at least one glucocorticoid.

### Brief Description of the Figures

- **Figure 1a**:: High resolution computer tomography (HR-CT) of the lung of a 58 year old patient. Severe lung fibrosis can be seen due to a chronic interstitial inflammatory process in hypersensivity pneumonitis prior to treatment with interferon-γ.
- **Figure 1b**:: HR-CT imaging of the lung of the same patient as in Fig. 1. Significant reduction of interstitial scarring after 12 months of treatment with interferon-γ has been visible.
- **Figure 2a**:: HR-CT imaging of a 28 year old patient suffering from chronic sarcoidosis prior to treatment with interferon-γ. Increased interstitial scarring and ground glass phenomena can be observed in both lungs.
- **Figure 2b**:: HR-CT imaging of the same patient as indicated in Fig. 2a. six months after treating with interferon-γ. The dramatic reduction of fibrotic lesions and ground glass phenomena can be seen in both lungs as a sign of decreased interstitial inflammation.
- **Figure 3a:**: Measurement of the total lung capacity (TLC) prior and after treatment of several patients with interferon-γ (200 µg /dose, 3 times per week, subcutaneously) and prednisolone (10 mg/dose per week, orally). TLC was determined by body plethysmography. Absolute values were given at visit 1 (0) (after an initial therapy with 10 mg prednisolone for at least 4 weeks) and after 9 months of the combination therapy. A significant improvement of lung functions can be observed.
- **Figure 3b**:: Same therapy and measurements as pointed out in Fig. 3a, however with a interferon dosage of 100 µg. In contrast to the therapy with 200 µg/dose no improvement of lung function was observed. Only 3 of 6 patients showed a constant TLC, the other 3 patients presented a decreased TLC comparable with the situation prior to treatment.

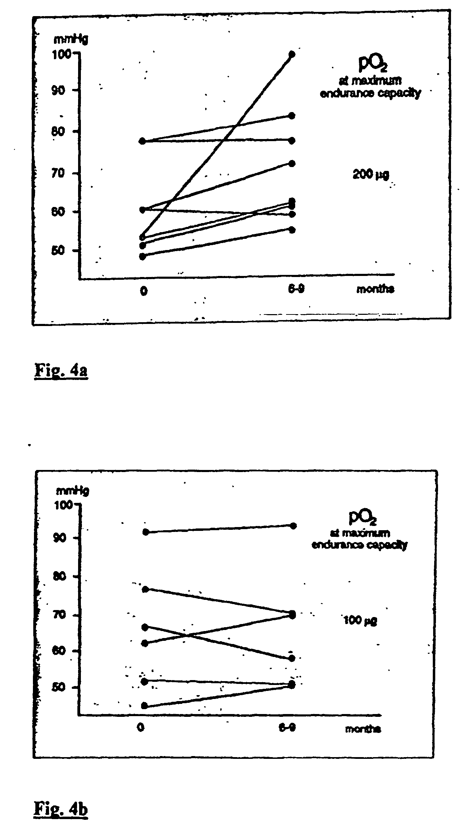

- **Figure 4a:**: Effect of the combined therapy with interferon-γ (200 µg /dose, 3 times per week, subcutaneously) and prednisolone (10 mg/dose per week, orally) on the arterial oxgen concentration (paO₂) at maximum endurance capacity after 6-9 months of therapy. Patients performed ergometry on a bicycle ergometer in two-minutes steps until maximum endurance capacity was reached. Arterial blood gases were taken at every step. In contrast to the seady decrease observed in patients receiving only 10 mg prednisolone alone, all patients (including those who had severely diminished lung function prior to the treatment with interferon) showed a significant improvement of the arterial oxygen concentration.
- **Figure 4b**:: Same combination therapy and measurements as described in Fig. 4a, however with 100 µg interferon-γ only. Only 3 of 6 patients showed an improvement of oxygen uptake during exercise, whereas the other 3 patients had a decrease of oxygenation.

### Detailed Description of the Invention

Patients suffering from different forms of interstitial pulmonary fibrosis such as idiopathic fibrosis, exogen allergic alveolitis, sarcoidosis or scleroderma were included with regard to anamnestic signs of proliferative lung disease. In order to allow the comparison of lung function within the study group patients suffering from acute interstital inflammation as well as patients with end-stage fibrosis were excluded. Nevertheless, it must be pointed out that the invention can be used also for patients with said starting and end characteristics and symptoms, respectively.
Functional assessment consists of measurement of total lung capacity (TLC), inspiratory vital capacity (IVC), functional exspiratory vital capacity (FVC), residual lung volume (RV), peak exspiratory flow (PEF), D_{L}CO and alveolar-arteriolar oxygen difference (AaDO₂) as well as of the assessment of paO₂ and paCO₂ under conditions of standardized ergometry. These examinations were preferably performed at the beginning of the observation period on day 1 of treatment, after one month of therapy, and afterwards every three months during the whole observation period of one year.

Roentgenographic examination by high resolution computer tomography (HR-CT) was performed prior to the application of interferon-γ after one month of therapy, and then every three months until the end of the observation period. Moreover, assessment of the inflammatory response was performed by serological investigation during the whole observation period as well as by histological and molecular analysis according to known standard techniques of several transbronchial biopsies withdrawn prior to treatment and after at least 6 months of therapy with interferon-γ. Bronchoscopy and transbronchial biopsies were only performed in patients who explicitly gave informed consent for this investigation. In addition, the expression of the TGF-β gene was measured in several cases using the known quantitative reverse transcriptase-polymerase chain reaction (qRT-PCR). Total RNA was isolated from transbronchial specimens taken identically during bronchoscopy prior to treatment with interferon-γ and after six months of treatment, respectively. The lung segments chosen for investigation were determined by HR-CT according to standard techniques. In addition, a standardized questionnaire for assessment of life quality and physical condition was implemented.

In a special trial of the invention 30 patients were included into the combination therapy (interferon-γ + glucocorticoid). Group 1 received glucocorticoid treatment (about 10 mg daily) alone. Group 2 received the same glucocorticoid treatment in combination with interferon-γ at lower dosages (between 70 -120 µg, preferably 100 µg) and group 3 reveived the same glucocorticoid treatment with interferon-γ at higher dosages (between 170 and 250 µg, preferably 200 µg). Interferon was administered preferably 3 times per week.

**Table 1**

| *Base-line characteristics of the patients; histological assessment and duration of symtoms.* | | |
|---|---|---|
| | number | time after diagnosis (years) |
| Sarcoidosis | 13 | 1-6 |
| Hypersensitivity pneumonitis | 8 | 2-9 |
| Idiopathic lung fibrosis | 7 | 3-10 |
| Lung involvement in scleroderma | 2 | 6 and 20 |

**Table 2**

| *Gender and age distribution within the treatment groups.* | | | | |
|---|---|---|---|---|
| | number | men | women | age |
| prednisolone alone | 10 | 6 | 4 | 45.9 ± 11.0 |
| prednisolone + 100 µg IFN | 10 | 4 | 6 | 47.6 ± 12.4 |
| prednisolone + 200 µg IFN | 10 | 6 | 4 | 47.5 ± 12.1 |

Of course, also different and alternative examination and observation conditions /techniques of the prior art, time schedules and dosage intervals are possible according to the invention.

Apart from the special trial described above, the dosage of interferon-γ varies according to the invention from 70 - 280 µg per single dose, preferably from 100 - 220 µg, more preferably from 175 - 200 µg. The most preferred dosage is about 200 µg. Calculated at kg body weight, the following amounts of interferon-γ can be used: 1 - 3.5 / 4.0 µg / kg / single dose, preferably 1.4 - 3.2 µg / kg / single dose, more preferably 2.0 - 3.0 µg / kg / single dose, and most preferably 2.5 - 2.8 µg / kg / single dose.

The above-indicated single dosages of interferon-γ are administered parenteral, preferably subcutaneously to the patient three times per week. Thus, a weekly dosage for a patient is between approximately 210 and 840 µg interferon.

However, it is possible to distribute this weekly dosage among more than three applications. Less than three applications of the same dosage in a week are not recommendable since the known side effects are too strong.

If interferon-γ is applied not together with glucocorticoids the weekly dosage is preferably somewhat reduced in order to avoid severe side effects. As supportive therapy drugs that diminish said side effects, e.g. antipyretics such as paracetamol or salicylate in usual dosages can additionally be administered.

In the combination therapy according to the invention glucocorticoids additionally to inerferon-γ are applied. All synthetic or natural glucocorticoids which are suitable for pharmaceutical applications can be used. Many of them are commercially available. Examples for suitable glucocorticoids are hydrocortison, cortison, dexamethason, betamethason, prednisolone, methyl prednisolone and their non-toxic salts. The preferred glucocorticoid according to the inventiom is prednisolone and salts thereof.

Also combinations and mixtures of several glucocorticoids are applicable, if necessary. The doses of glucocorticoids which should be administered to a patient vary according to the invention from 5 - 25 mg / single dose and more preferably from 10 - 15 mg. The most preferred dosage is about 10 mg. Calculated at kg body weight, glucocorticoid doses between 100 and 350 µg / kg / single dose are preferred. Preferably, this dosage is given daily which means that the weekly dosage is between 35 and 175 mg. According to the invention, also somewhat smaller or higher dosages are applicable without obtaining a changed therapeutical effect. Preferably, the glucocorticoids are applied orally. However, also parenteral applications may be used.

In a preferred embodiment of the invention the glucocorticoid therapy using the same dosages as mentioned above, is initiated approximately four weeks before the interferon application is started . However, it is possible to prolong or to shorten the period of said pre-administration of glucocorticoids. The initial dosage may be higher than the long-term dosage.

Analysis of transcription of the transforming growth factor-β (TGF-β) gene in transbronchial ling biopsies of several patients by qRT-PCR before and after six months of treatment with gamma-interferon reveales an induced transcription of TGF-β ranging from 0.1 to 1 attomole (amole) in all patients even after an initial glucocorticoid treatment, whereas in lung biopsies from healthy persons no transcription of TGF-β can be found. The combination of glucocorticoid and IFN-gamma (200 µg) consistently causes a decrease of TGF-β gene transcription by factor 10.

In general, the optimum therapeutically acceptable dosage and dose rate for interferon-γ and glucocorticoids for a given patient within the above-said ranges depends on a variety of factors, such as the activity of the specific active material employed, the age, body weight, general health, sex, diet, time and route of administration, rate of clearance or the object of treatment.

The term "parenteral" as mentioned above and below includes subcutaneous, intravenous, intra-articular and intratracheal injection and infusion techniques. Oral administration is applicable only in the case of glucocorticoids. This application is not suitable for polypeptides like interferones since they are not bio-available after passing the gastrointestinal tract.

It is a prefered embodiment of the invention to use both drugs seperated as individual pharmaceutical compositions within the combination therapy. Because of that, different and variable times of administration are allowable, if necessary. This can also be achieved by providing a medical kit-of-parts in which separated but well defined pharmaceutical formulations adjusted to each other, and comprising interferon-γ (on the one hand), and the glucocorticoid (on the other hand), in concentration and dosages, as indicated above, are available as a medical unit.

However, it is also possible to provide a single pharmaceutical composition comprising interferon-γ in a suitable concentration together with a glucocorticoid in a suitable concentration, and together with a suitable pharmaceutically acceptable carrier or excipient that allows a standardized soluble formulation suitable for parenteral administration.

As used herein, the term "pharmaceutically acceptable carrier or excipient" means an inert, non toxic liquid filler, diluent, solvent or solution, not reacting adversely with the active compounds or with the patient. Suitable liquid carriers are well known in the art such as steril water, saline, aqueous dextrose, sugar solutions, ethanol, glycols and oils, including those of petroleum, animal, vegetable, or synthetic origin. The formulations may also contain adjuvants or vehicles which are typical for parenteral administration.

With respect to said suitable formulations it should be pointed out that interferon-γ and glucocorticoids may eventually form pharmaceutically acceptable salts with any non-toxic, organic or inorganic acid showing changed solubility. Inorganic acids are, for example, hydrochloric, sulphuric or phosphoric acid and acid metal salts such as sodium monohydrogen orthophosphate and potassium hydrogen sulfate. Examples for organic acids are the mono, di and tri carboxylic acids such as acetic, glycolic, lactic, pyruvic, malonic, succinic, glutaric, fumaric, malic, tartaric, citric, ascorbic, maleic, benzoic, phenylacetic, cinnamic, salicylic and sulfonic acids. Salts of the carboxy terminal amino acid moiety include the non-toxic carboxylic acid salts formed with any suitable inorganic or organic bases. These salts include, for example, alkali metals such as sodium and potassium, alkaline earth metals such as calcium and magnesium, and organic primary, secondary and tertiary amines such as trialkylamines.

A typical liquid pharmaceutical composition for the combination therapy according to the invention is administered daily by parenteral techniques, and comprises, for example, interferon-γ in a dosage of 70 - 100 µg / single dose together with 10 mg glucocorticoid / single dose. However, analogously adapted pharmaceutical formulation which are adminstered each second or third day are also usable according to the invention.

Therapy with glucocorticoid treatment alone does not improve lung volumina or exercise performance. On the contrary, during the 12-months observation period, patients in this group show a steady decrease of lung function and gas diffusion both at rest and during exercise conditions.

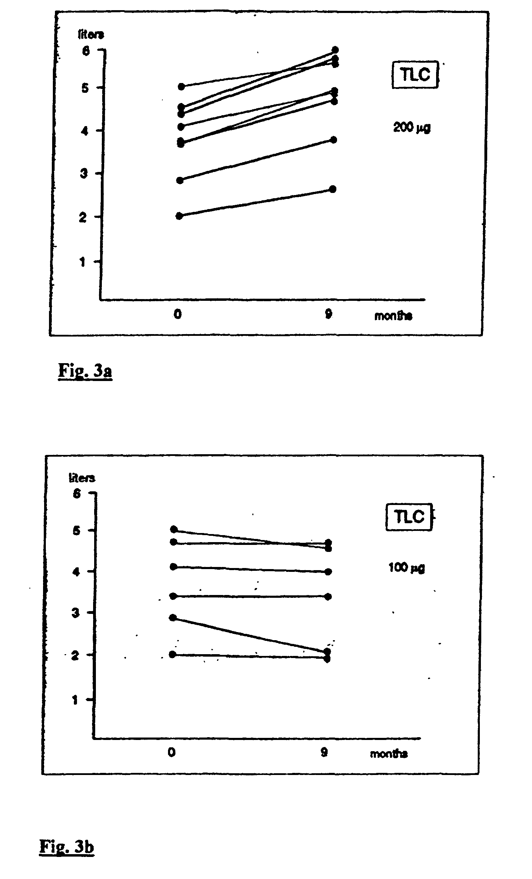

The results of an additional therapy regimen with interferon-γ with increasing doses show that interferon-γ at lower doses (which are usually administered in the known therapies) has no or only a reduced effect. The effect of interferon-γ (100 and 200 µg, 3 times per week) on TLC, the most reliable static lung volume with regard to interstitial lung diseases is given in Figure 3a, b. Whereas the addition of 100 µg interferon did not show an improvement of TLC, 200 µg interferon cause a clear increase of the total lung capacity in all patients, even in patients with long-term disease and a severely diminished lung function.

These findings become clearer when the oxygen uptake under maximum exercise conditions is compared. Again, the group of patients receiving a higher dosage of interferon than usual (200 µg) show the best results with a sometimes dramatic improvement already after six months of therapy (Figure 4a, b). But also nearly half of the patients who were treated with lower interferon-γ doses (100 µg) has an improvement of gas exchange under exertion which is the most sensitive way of addressing a compromised diffusion capacity in interstitial lung diseases.

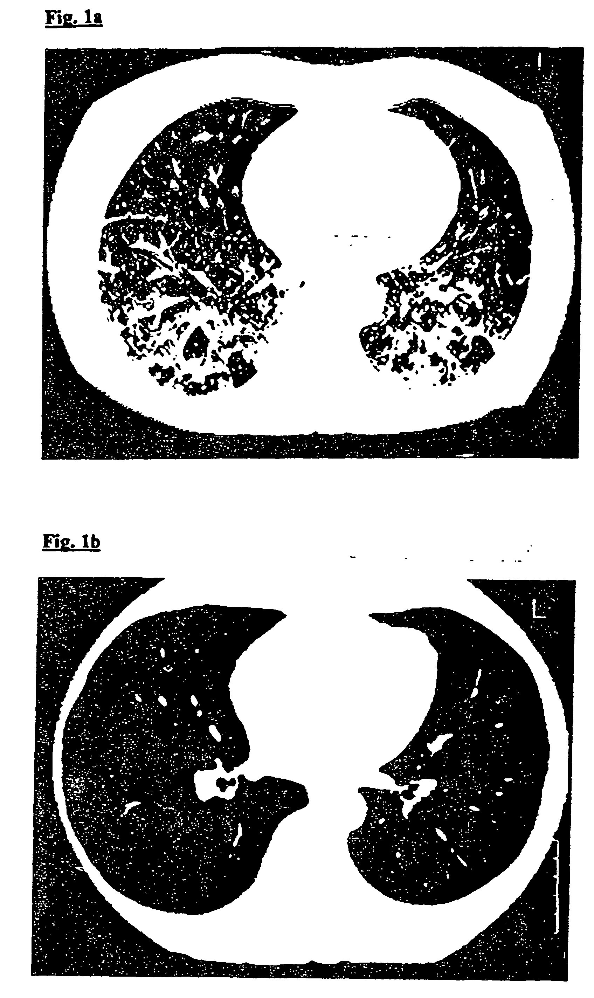

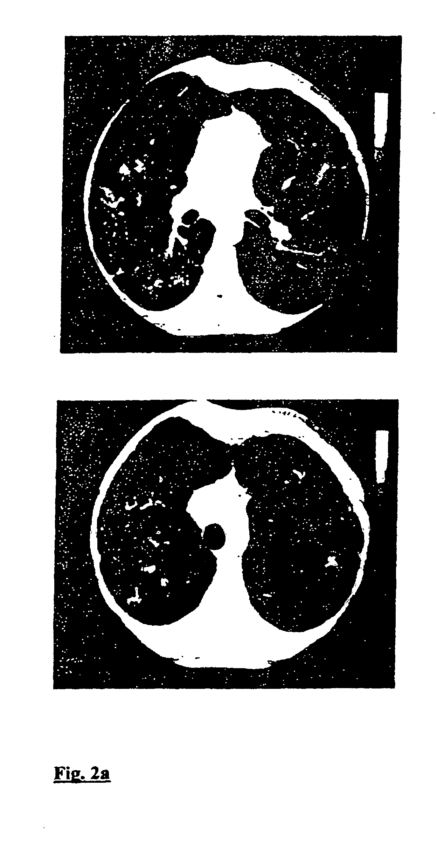

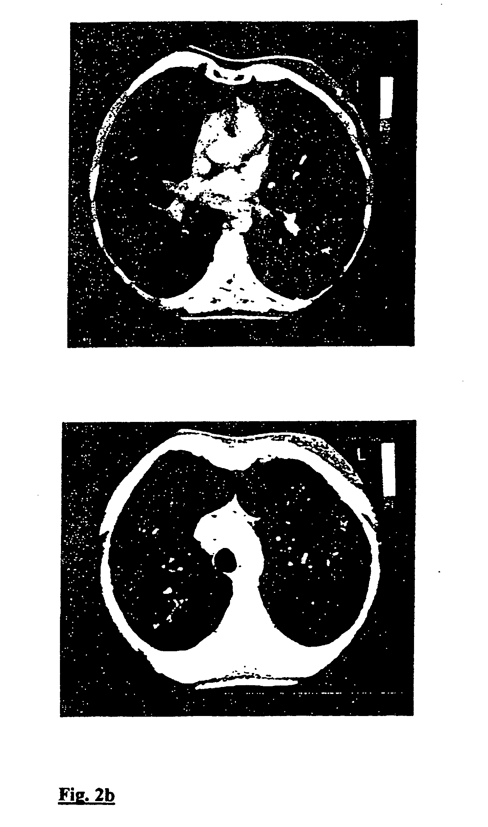

Morphological assessment of lung structure by HR-CT confirms impressively these functional results (Figure 1a, b; Figure 2a, b). Histological evaluation which was performed in patients who gave informed consent to an additional evaluation by transbronchial biopsy yields additional proof that treatment with interferon-γ according to the invention is able to diminish and even reverse inflammation and fibrosing alveolitis in chronic lung diseases.

## Claims

1. Use of interferon-γ for the manufacture of a medicament for the treatment of interstitial lung diseases, wherein the single dose of interferon-γ in said medicament varies between 1.0 - 3.5 µg / kg bodyweight.

2. Use of interferon-γ according to claim 1 for the treatment of all forms of interstitial pulmonary fibrosis.

3. Use of interferon-γ according to claim 1 or 2 for the treatment of long-term interstitial lung diseases.

4. Use of interferon-γ according to claims 1 - 3 together with at least one glucocorticoid.

5. Use of interferon-γ according to claim 4, wherein the single dose of the glucocorticoid varies from 100 - 350 µg / kg bodyweight, preferably from 100 -150 µg / kg bodyweight.

6. Pharmaceutical composition comprising interferon-γ, at least one glucocorticoid and, optionally, a pharmaceutically acceptable carrier and/or excipient, wherein the single dose of interferon-γ varies between 70 - 280 µg, and the single dose of the glucocorticoid varies from 5 - 25 mg.

7. Kit of parts suitable for the treatment of interstitial lung diseases comprising a first pharmaceutical composition comprising interferon-γ and a second pharmaceutical composition comprising at least one glucocorticoid, each optionally together with a pharmaceutically acceptable carrier and/or excipient, wherein the single dose of interferon-γ varies from 70 - 280 µg.

8. Pharmaceutical composition according to claim 6, wherein the single dose of interferon-γ varies from 100 - 220 µg.

9. Pharmaceutical composition according to claim 6, wherein the single dose of interferon-γ varies from 175 - 200 µg.

10. Pharmaceutical composition according to claim 6, wherein the single dose of glucocorticoid varies from 10 - 15 mg.

## Patentansprüche

1. Verwendung von Interferon-γ zur Herstellung eines Medikaments für die Behandlung von interstitiellen Lungenerkrankungen, wobei die Einzeldosis von Interferon-γ in dem Medikament von 1,0 bis 3,5 µg/kg Körpergewicht variiert.

2. Verwendung von Interferon-γ nach Anspruch 1 für die Behandlung aller Formen interstitieller Lungenerkrankungen.

3. Verwendung von Interferon-γ nach Anspruch 1 oder 2 für die Behandlung langfristiger interstitieller Lungenerkrankungen.

4. Verwendung von Interferon-γ nach den Ansprüchen 1 bis 3, gemeinsam mit mindestens einem Glucocorticoid.

5. Verwendung von Interferon-γ nach Anspruch 4, wobei die Einzeldosis des Glucocorticoids von 100 bis 350 µg /kg Körpergewicht, vorzugsweise von 100 bis 150 µg/kg Körpergewicht variiert.

6. Pharmazeutische Zusammensetzung, umfassend Interferon-γ, mindestens ein Glucocorticoid und wahlweise einen pharmazeutisch annehmbaren Träger und/oder ein Vehikel, wobei die Einzeldosis von Interferon-γ von 70 bis 280 µg variiert und die Einzeldosis von Glucocorticoid von 5 bis 25 mg variiert.

7. Komponentensatz, der für die Behandlung interstitieller Lungenerkrankungen geeignet ist, umfassend eine erste pharmazeutische Zusammensetzung, umfassend Interferon-γ, und eine zweite pharmazeutische Zusammensetzung, umfassend mindestens ein Glucocorticoid, jeweils wahlweise gemeinsam mit einem pharmazeutisch annehmbaren Träger und/oder Vehikel, wobei die Einzeldosis von Interferon-γ von 70 bis 280 µg variiert.

8. Pharmazeutische Zusammensetzung nach Anspruch 6, wobei die Einzeldosis von Interferon-γ von 100 bis 220 µg variiert.

9. Pharmazeutische Zusammensetzung nach Anspruch 6, wobei die Einzeldosis von Interferon-γ von 175 bis 200 µg variiert.

10. Pharmazeutische Zusammensetzung nach Anspruch 6, wobei die Einzeldosis des Glucocorticoids von 10 bis 15 mg variiert.

## Revendications

1. Utilisation de l'interféron-γ pour la fabrication d'un médicament destiné au traitement des maladies interstitielles pulmonaires, selon laquelle la dose unique de l'interféron dudit médicament se trouve comprise entre 1,0 et 3,5 µg / kg du poids corporel.

2. Utilisation de l'interféron-γ selon la revendication 1 pour le traitement de toutes les formes de fibroses interstitielles pulmonaires.

3. Utilisation de l'interféron-γ selon la revendication 1 ou 2 pour le traitement des maladies interstitielles pulmonaires à long terme.

4. Utilisation de l'interféron-γ selon les revendications 1 à 3 avec au moins un glucocorticoïde.

5. Utilisation de l'interféron-γ d'après la revendication 4, selon laquelle la dose unique de glucocorticoïde se trouve comprise entre 100 et 350 µg / kg du poids corporel, de préférence entre 100 et 150 µg / kg du poids corporel.

6. Composition pharmaceutique comprenant l'interféron-γ, au moins un glucocorticoïde et, éventuellement un support et/ou un excipient acceptable sur le plan pharmaceutique, selon laquelle la dose unique d'interféron-γ se trouve comprise entre 70 et 280 µg / kg du poids corporel, et la dose unique du glucocorticoïde se trouve comprise entre 5 et 25 mg.

7. Kit composé des parties convenables pour le traitement des maladies interstitielles pulmonaires contenant une première composition pharmaceutique comprenant l'interféron-γ et une seconde composition pharmaceutique comprenant au moins un glucocorticoïde, chacun étant éventuellement accompagné d'un support et/ou excipient acceptable sur le plan pharmaceutique, et selon lequel la dose unique d'interféron-γ se trouve comprise entre 70 et 280 µg.

8. Composition pharmaceutique d'après la revendication 6, selon laquelle la dose unique d'interféron-γ se trouve comprise entre 100 et 220 µg.

9. Composition pharmaceutique d'après la revendication 6, selon laquelle la dose unique d'interféron-γ se trouve comprise entre 175 et 200 µg.

10. Composition pharmaceutique d'après la revendication 6, selon laquelle la dose unique de glucocorticoïde se trouve comprise entre 10 et 15 mg.
